Europäisches Patentamt

(19) European Patent Office

Office européen des brevets

(11) EP 0 553 376 B1

(12) **EUROPEAN PATENT SPECIFICATION**

(45) Date of publication and mention
of the grant of the patent:
**10.01.1996 Bulletin 1996/02**

(51) Int. Cl.$^6$: **C07C 31/36**, C07C 29/62

(21) Application number: **92101470.0**

(22) Date of filing: **29.01.1992**

(54) **Process for producing 2,3-dichloro-1-propanol and reactor used therefor**

Verfahren zum Herstellen von 2,3-Dichlor-1-propanol und dafür verwendeter Reaktor

Procédé pour la production du 2,3-dichloro-1-propanol et réacteur utilisé

(84) Designated Contracting States:
**DE FR GB NL**

(43) Date of publication of application:
**04.08.1993 Bulletin 1993/31**

(73) Proprietor: **SHOWA DENKO KABUSHIKI KAISHA
Minato-ku, Tokyo (JP)**

(72) Inventors:
• **Nojima, Hiromitu
Kawasaki-shi, Kanagawa (JP)**
• **Ohki, Yoshihiro
Kawasaki-shi, Kanagawa (JP)**
• **Mori, Toshio
Kawasaki-shi, Kanagawa (JP)**

• **Kudo, Koji
Kawasaki-shi, Kanagawa (JP)**
• **Shiroiwa, Yasushi
Kawasaki-shi, Kanagawa (JP)**

(74) Representative: **Henkel, Feiler, Hänzel & Partner
D-81675 München (DE)**

(56) References cited:
**DE-A- 2 527 630          DE-A- 3 006 791
FR-A- 2 565 229          GB-A- 1 342 319
US-A- 4 788 351**

• **PATENT ABSTRACTS OF JAPAN vol. 8, no. 251
(C- 252)(1688), 16 November 1984; & JP - A -
59128340 (SHOWA DENKO K.K.) 24.07. 1984**

**Description**

This invention relates to a process for producing 2,3-dichloro-1-propanol by reacting allyl alcohol dissolved in hydrochloric acid with chlorine in a high yield while inhibiting formation of by-products, and to a falling film type reactor for carrying out the chlorination reaction of allyl alcohol with good efficiency.

BACKGROUND OF THE INVENTION

2,3-dichloro-1-propanol (hereafter referred to as 2,3DCP) is a useful compound as a solvent and an intermediate for production of epichlorohydrin widely used, a starting material for synthetic rubbers and epoxy resins, and a stabilizer for chlorinated rubbers.

In production of 2,3DCP by chlorination reaction of allyl alcohol, allyl alcohol is dissolved in a solvent, and the dissolved allyl alcohol is chlorinated.

Hitherto, a stirred vessel-type reactor has been used for the chlorination reaction (US-A-4778351). But, since heat of reaction generated in chlorination of allyl alcohol is as high as about 60 kcal/mol and a jacket provided in the stirred vessel has an insufficient cooling surface, an external heat exchanger having a large heat transfer surface area is provided for cooling and the temperature is adjusted by circulating the reaction liquid between the stirred vessel and the external heat exchanger.

As a solvent used in the chlorination of allyl alcohol, an organic solvent and hydrochloric acid are known, as described in JP-A-46-1361, JP-B-48-18207, JP-B-37-17206 and U.S. Patent 4,634,784. (The terms "JP-A" and "JP-B" mean an unexamined published Japanese patent application and an examined published Japanese patent application, respectively.)

In a method described in U.S. Patent 4,634,784 hydrochloric acid having a hydrogen chloride concentration of 45 to 70% by weight is used as a solvent, this method is excellent in that the yield of 2,3DCP, based on ally] alcohol, is more than 90%. However, amounts of some by-products increases as the hydrogen chloride concentration is increased, and a countermeasure for inhibiting the formation of by-products has not be reported.

In chlorination of allyl alcohol to produce 2,3DCP, monochlorohydrin is produced as a by-product due to the reaction of hypochlorous acid with allyl alcohol as shown in scheme (1):

$$CH_2=CH \cdot CH_2OH + HClO \rightarrow CH_2Cl\text{-}CH_2\text{-}CH_2OH + CH_3\text{-}CHCl\text{-}CH_2OH \qquad (1)$$

Hypochlorous acid has an equilibrium relation (2):

$$Cl_2 + H_2O \rightleftarrows HClO + HCl \qquad (2)$$

Accordingly, when the concentration of hydrogen chloride in the hydrochloric acid used as the solvent is increased to inhibit the formation of hypochlorous acid, the formation of monochlorohydrin can be inhibited, and the yield of 2,3DCP can be improved.

By increasing the hydrogen chloride concentration in hydrochloric acid, the formation of by-products such as monochlorohydrin is inhibited, but in turn, amounts of reaction products with chlorine, such as ally] chloride and 1,2,3-trichloropropane, tend to increase. It is also desired to inhibit formation of these by-products.

SUMMARY OF THE INVENTION

It is an object of this invention to provide a process for producing 2,3DCP in a high yield by chlorinating allyl alcohol while inhibiting formation of by-products, and also to provide a reactor suitable for the process.

The present inventors made extensive investigations to solve the above problem, and it has been found that the amount of monochlorohydrin, etc., whose formation can be inhibited by using hydrochloric acid having a high concentration, is not dependent on the residence time of reaction liquid in the reactor, whereas the amounts of by-products due to a reaction with chlorine, such as 1,2,3-trichloroproane, etc., whose formation becomes remarkable as the hydrochloric acid concentration is higher, increases almost in proportion to the residence time of reaction liquid in a reactor. The present invention has been made on the above finding.

In the process for producing 2,3DCP of this invention, allyl alcohol dissolved in hydrochloric acid is reacted with chlorine using a falling film type reactor to solve the problem.

BRIEF DESCRIPTION OF THE DRAWINGS

Figs. 1 and 2 show one example of the reactor for practicing the process of this invention. Fig. 1 is an entire view, and Fig. 2 is an enlarged view of the portion (a) of Fig. 1.

Figs. 3 to 7 show a preferred example of the reactor used in this invention. Fig. 3 is a longitudinal sectional view, and Fig. 4 shows arrangement of heat transfer tubes in the cross-sectional view at the portion II-II as indicated with arrows in Fig. 3). Fig. 5 is an enlarged view of portion A of Fig. 3. Fig. 6 is an enlarged view of portion B of Fig. 3. Fig. 7 is a cross-sectional view of a liquid distribution pipe having holes of Fig. 5.

Fig. 8 is an explanatory view of the reactor of Fig. 3 as it is used.

DETAILED DESCRIPTION OF THE INVENTION

Since a falling film type reactor is employed in this invention, the residence time of reaction liquid in the reactor can be very short, generally not longer than one hour, preferably within 20 minutes, and the heat transfer surface area with respect to the amount of the reaction liquid residing in the reactor can be increased. Further, hydrochloric acid having the hydrogen chloride concentration as high as 20 wt% or more, preferably from 45 to 75 wt% can be used as a solvent of allyl alcohol by holding the reaction liquid under pressurized state, generally from 0 to 10 $kg/cm^2$, preferably from 0.5 to 3 $kg/cm^2$. Thus, 2,3DCP can be effectively produced in a high yield while inhibiting the formation of by-products such as monochlorohydrin, allyl chloride, and 1,2,3-trichloropropane.

The residence time (hour) is calculated by dividing the amount ($m^3$) of reaction liquid held in the reactor by flow rate ($m^3$/hour) of the liquid.

In the falling film type reactor used in this invention, usually a plurality of tubes are provided perpendicularly, a starting material liquid composed of allyl alcohol dissolved in hydrochloric acid of a high hydrogen chloride concentration is allowed to flow down through inner walls of the perpendicular tubes with a parallel flow of chlorine or chlorine diluted with hydrogen chloride, and a refrigerant is passed over the outside of the tubes for cooling.

The reaction temperature is -20 to 20°C and preferably 0 to 10°C. Therefore, the temperature of the refrigerant is further lower.

The chlorination reaction proceeds very rapidly, and therefore, a specific device is required so as not to locally generate heat in the upper portion of the reactor, ensuring that a chlorine gas is reacted uniformly within the tube.

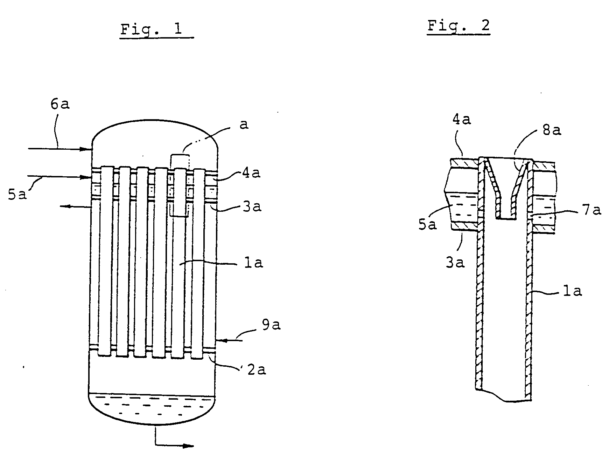

The falling film type reactor which can be used in the process of this invention is briefly explained with reference to Figs. 1 and 2, wherein 1a is a falling film tube; 2a a lower tube sheet; 3a an upper first tube sheet; 4a an upper second tube sheet; 5a a starting material solution; 6a chlorine gas; 7a a hole; 8a an orifice; and 9a a refrigerant.

A plurality of falling film tubes 1a are held perpendicularly with a lower tube sheets 2a, upper first tube sheet 3a and an upper second tube sheet 4a spaced from each other, and a starting material solution 5a composed of a hydrochloric acid solution of allyl alcohol is introduced between the upper first tube sheet 3a and the upper second tube sheet 4a. Furthermore, chlorine gas 6a is introduced into a portion above the upper second tube sheet 4a.

The starting material solution 5a flows inside the tube 1a from the hole 7a provided in the falling film tube 1a, and flows down in the form of a thin film through the tubular wall. On the other hand, chlorine gas is uniformly distributed in each of the tubes by the orifice 8a provided above the tube 1a, and comes down as a parallel flow with the starting material solution 5a and chlorination takes place. The reaction proceeds mainly in that part which is cooled outside by the refrigerant 9a, and the reaction temperature does not rise locally. The reaction speed is fast, and the reaction is completed at the lower end of the tube 1a. The residence time of reaction liquid in the reactor is very short as compared with that using a conventional stirred vessel-type.

Chlorination of allyl alcohol is a very rapid reaction as mentioned above, and it is required to inhibit the reaction from locally taking place at the upper portion of the reactor. Furthermore, the thickness of a reaction liquid film flowing down the wall surface of the falling film type reactor should be reduced. Accordingly, to increase the amount of the starting material solution to be reacted, the diameter of the falling film type reactor should be increased to enlarge the flow-down area of the solution. But this results in reduction of the falling film area per installation area of the reactor.

The present inventors found that the above inconvenience can be dissolved by using a falling film type reactor having the following structure.

That is, as another embodiment of this invention, there is provided a reactor for producing 2,3DCP with good efficiency, which comprises:

(i) a cylindrical shell having (a) nozzles for introducing and discharging a refrigerant provided in lower and upper portions of the shell, (b) horizontal upper and lower tube sheets, and (c) a plurality of vertical heat transfer tubes provided between the upper and lower tube sheets, through which a portion above the upper tube sheet is connected to a portion beneath the lower tube sheet;
(ii) a lower cover beneath the shell, having provided therein (d) a nozzle for introducing and discharging a reaction liquid; and
(iii) an upper cover above the shell, having provided therein (e) a horizontal restriction plate separating the upper cover into a lower liquid reservoir having a liquid-introducing nozzle and an upper gas reservoir having a gas-introducing nozzle;

wherein the upper surface of the upper tube sheet is provided with (f) liquid distribution pipes located on the extension of the heat transfer tubes, the liquid distribution pipes having holes in the vicinity of the upper surface of the upper tube sheet to allow a liquid in the liquid reservoir to come into the heat transfer tubes and having an upper end opening close to the restriction plate which has formed therein an orifice hole facing toward the upper end opening of the liquid distribution pipe to allow a gas in the gas reservoir to be uniformly distributed in each of the heat transfer tubes.

Since the reactor is so constructed as above, allyl alcohol dissolved in hydrochloric acid and chlorine gas can be uniformly distributed and introduced inside the tube, so that the reaction proceeds at a low temperature with efficient cooling of the reaction system.

That is, chlorine gas is maintained at a constant pressure in the gas reservoir, and fed into a heat transfer tube through an orifice provided in the restriction plate hole. Accordingly, the amount of chlorine fed to each of the heat transfer tubes is constant.

On the other hand, the starting material solution maintained in the liquid reservoir at a predetermined level of liquid surface is introduced into the liquid distribution pipes through the holes of the pipes and a gap is formed between the upper end opening of each of the liquid distribution pipes and the lower surface of the restriction plate. Accordingly, the difference in pressure between the inside and outside of the liquid distribution pipes becomes constant and the solution is introduced uniformly into each of the liquid distribution pipes. In addition, since chlorine gas is ejected into the liquid distribution pipes through the orifice hole, chlorine gas hardly flows into the liquid reservoir through the gap.

In view of use of highly corrosive starting materials for production of 2,3DCP, the reactor must be formed of an anti-corrosive material. For the purpose, a graphite and a glass lining which are widely used in an apparatus for producing hydrochloric acid are used in this invention.

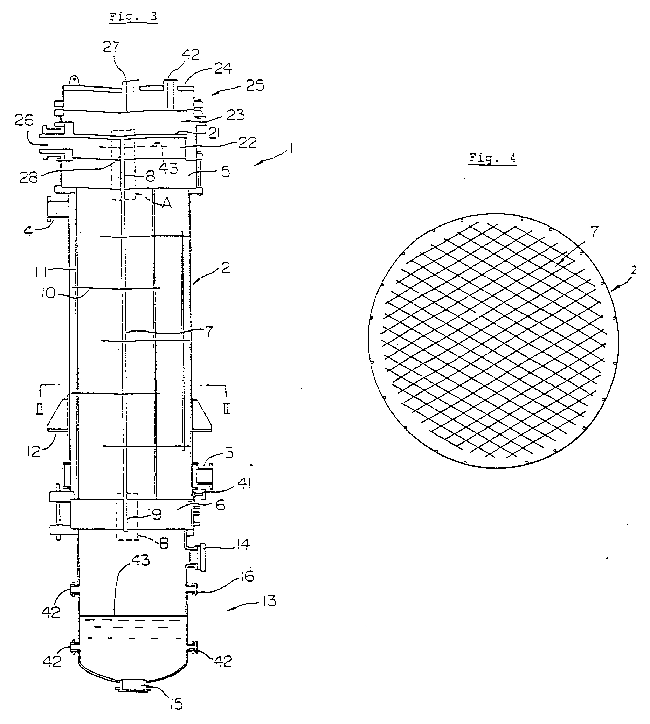

Figs. 3 to 7 show one example of a preferred rector suitable for production of 2,3DCP, wherein 1 is a chlorination reactor; 2 a cylindrical shell; 3 a refrigerant-introducing nozzle; 4 a refrigerant-discharging nozzle; 5 an upper tube sheet; 6 a lower tube sheet, 7 a heat transfer tube; 8 an upper hole; 9 a lower hole; 10 a baffle plate; 11 a baffle plate stay; 12 a supporting leg; 13 a lower cover; 14 a gas-discharging nozzle; 15 a liquid-discharging nozzle; 16 a starting material-introducing nozzle; 21 a restriction plate; 22 a liquid reservoir; 23 a gas reservoir; 24 a lid; 25 an upper cover; 26 a reaction liquid-introducing nozzle; 27 a gas-introducing nozzle; 28 a liquid distribution pipe; 29 gap;, 30 an orifice hole; 31 a restriction plate hole, 32 a hole; 41 a drain nozzle; 42 a nozzle for instruments; 43 liquid surface; and 44 a pump.

The numeral 2 shows a cylindrical shell having a refrigerant-introduction nozzle 3 and a refrigerant-discharging nozzle 4. At the upper and lower opening portions of the shell 2, a horizontal upper tube sheet 5 and horizontal lower tube sheet 6 are provided. A number of heat transfer tubes 7 are fitted in the shell 2 as shown in Fig. 4 which is the cross-sectional view of the shell 2. The above upper tube sheet 5 and the lower tube sheet 6 have an upper hole 8 and a lower hole 9, respectively, which are connected through the heat transfer tubes 7 between the upper tube sheet 5 and the lower tube sheet 6.

The baffle plate 10 crossing perpendicularly with the heat transfer tubes 7 are supported by the baffle plate stay 11 and spaced therefrom. The supporting leg 12 for supporting the reactor perpendicularly is provided to the outside surface of the shell 2.

The lower cover 13 covering the lower tube sheet 6 is provided to the lower portion of the shell 2, and gas-discharging nozzle 14, a liquid-discharging nozzle 15 and a starting material-introduction nozzle 16 are provided in the lower cover 13.

A horizontal restriction plate 21 is provided in the upper portion of the shell 2 above the upper tube sheet 5 to divide it into a lower liquid reservoir 22 and an upper gas reservoir portion 23. The upper cover 25 sealed with a lid 24 is provided to form the gas reservoir 23. The liquid reservoir 22 is provided with a nozzle 26 for introducing the reaction liquid. To the lid 24 of the gas reservoir 23, is provided a gas-introducing nozzle 27 for introducing chlorine gas into the gas reservoir 23.

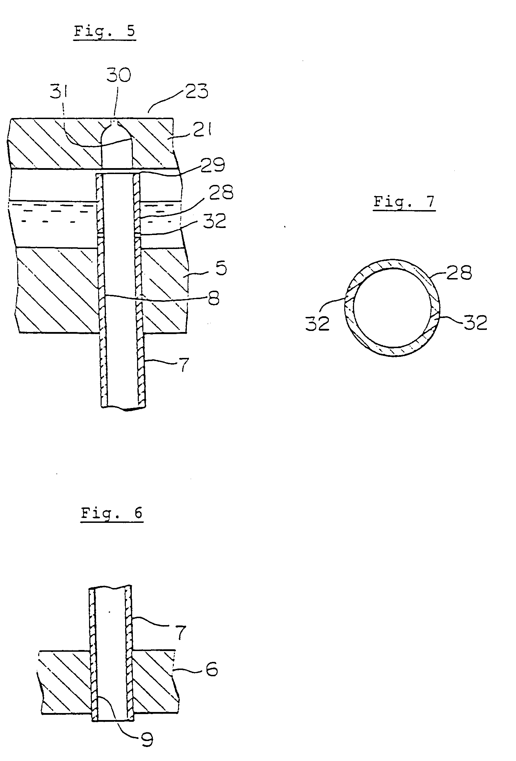

The enlarged views of portions A and B of Fig. 3 are shown in Figs. 5 and 6, respectively. To the upper hole 8 is provided the liquid distribution pipe 28 on the extension of the heat transfer tube 7. The upper end opening of the liquid distribution pipe 28 is close to the lower surface of the restriction plate 21 with a gap 29. The upper end opening of the liquid distribution pipe 28 faces a restriction plate hole 31 having an orifice hole 30 provided in the restriction plate 21. The orifice hole is not necessarily limited to that shown in Fig. 5 and may have any shape as long as it allows a gas in the gas reservoir to be uniformly distributed in each of the heat transfer tubes. As shown in Fig. 7, two holes 32 are provided in the liquid distribution pipe 28 in the vicinity of the upper surface of the upper tube sheet 5.

It is preferred that the holes 32 be provided in the liquid distribution pipes 28 as closely as possible to the upper surface of the upper tube sheet 5 and that the upper end openings of the liquid distribution pipes 28 be located as closely as possible to the restriction plate 28.

The numeral 41 is a drain nozzle, and 42 is a nozzle for instruments such as LA, LI, TA, TI and PIA.

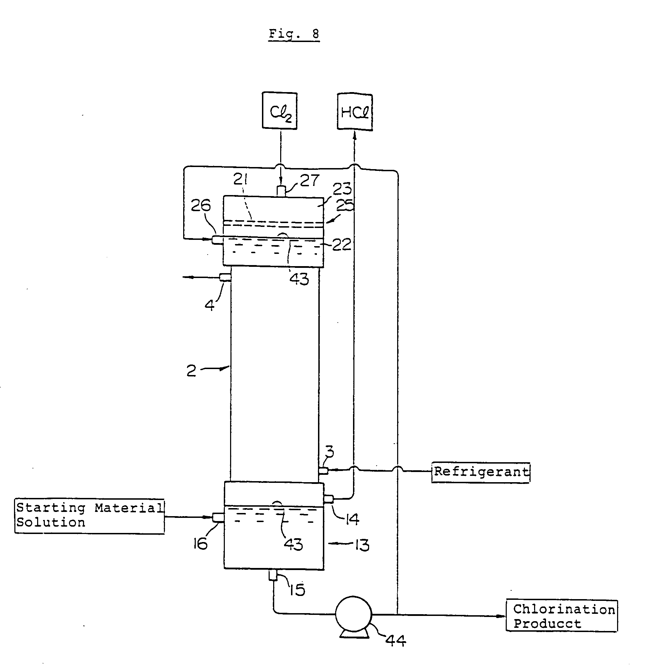

To perform chlorination of allyl alcohol using the above-mentioned reactor, as shown in Fig. 8, the starting material solution is introduced into the lower cover from the starting material-introducing nozzle 16 and then sent, by the pump 44, to the reaction solution-introducing nozzle 26 of the liquid reservoir 22. The reaction solution comes into the hole 32 of the liquid distribution pipe 28 and flows down to wet the inside wall of the heat transfer tube 7. Simultaneously, chlorine gas is introduced from the gas-introducing nozzle 27 and is uniformly distributed in the heat transfer tube 7 by the orifice hole of the restriction plate 21. The thus-distributed gas reacts with the reaction solution flowing down in the heat transfer tube 7. At this time, the flowing reaction solution is in the form of a thin film on the wall of the tube 7, and is cooled with a refrigerant (e.g., brine) from outside of the tube 7, and thus the tube 7 is efficiently cooled and maintained at a desired temperature. The chlorination reaction of allyl alcohol proceeds rapidly, and chlorine gas introduced into each of the heat transfer tubes 7 reacts with allyl alcohol and is consumed till it reaches at the lower cover. After the reaction reaches a steady state, the starting material solution is introduced while maintaining the liquid surface 43 in the liquid reservoir 22 and in the lower covered portion at a predetermined levels, and taking out 2,3DCP from the reactor.

The reaction liquid flowing down a plurality of heat transfer tubes 7 is prevented from being increased in temperature, and the chlorination proceeds with good efficiency. HCl formed by this reaction is taken out from the gas-discharging nozzle 14 and recovered.

Proceeding of the chlorination can be controlled by adjusting the amounts of the starting solution and chlorine gas introduced.

As stated above, the reactor preferably used in this invention utilizes a falling film wall composed of a number of heat transfer tubes of a small diameter. The starting solution can be introduced uniformly into each of the tubes to allow the liquid film to flow down, and as a parallel flow, chlorine gas can be uniformly introduced and reacted with the starting solution. Accordingly, the reactor can be made compact yet ensuring a large area of the falling film wall. Thus, the reactor of present invention makes it possible to perform the chlorination reaction for the production of 2,3DCP, which accompanies large heat generation, at a relatively low temperature with good efficiency. Economic advantages can be attained.

This invention is further explained in detail with reference to the following Examples but the invention is not limited thereto.

EXAMPLE 1

A refrigerant of -10°C was passed through the jacket of a Liebig condenser-type falling film type reactor having an inside diameter of 13 mm and a length of 1200 mm. At the same time, a 70 wt% aqueous solution of allyl alcohol cooled to -5°C by a refrigerant was saturated with hydrogen chloride, and the resulting solution was fed as a starting material solution from the vicinity of the top of the reactor at a flow rate of 126 g/hr. Chlorine gas to be reacted with allyl alcohol was fed at 85 g/hr so that the mole ratio of $Cl_2$ to allyl alcohol was about 1.1/1. The reaction liquid was taken out from the bottom portion of the reactor. Due to the exothermic reaction, the temperatures at the top and the bottom of the reactor were 5°C and 2°C, respectively. The amount of reaction liquid held in the reactor (including its bottom portion) was 300 ml.

After a steady state was reached, the liquid at the bottom portion was analyzed, revealing that conversion of allyl alcohol was 100%, yield of 2,3DCP was 93.5%, the amount of by-product monochlorohydrin was 3.7%, and the amount of by-product 1,2,3-trichloropropane was 0.5%.

COMPARATIVE EXAMPLE 1

A refrigerant of -10°C was passed through the jacket of a stirred-type reactor. Simultaneously, a 70 wt% aqueous solution of allyl alcohol cooled to -5°C was saturated with hydrogen chloride and the resulting solution was fed as a starting solution into the reactor at a flow rate of 126 g/hr. Chlorine gas to be reacted with allyl alcohol was fed into the reactor at 85 g/hr so that the mole ratio of $Cl_2$ to allyl alcohol was about 1.1/1. The reaction solution was taken out such that the amount of the liquid in the reactor became constant. The temperature of the inside of the reactor reached 5°C. The amount of the liquid in the reactor was 600 ml. After the reaction reached a steady state, the reaction solution was analyzed, revealing that conversion of allyl alcohol was 100%, yield of 2,3DCP was 91.5%, the amount of by-product monochlorohydrin was 3.7%, and the amount of by-product 1,2,3-trichloripropane was 1.0%.

EXAMPLE 2

Using the same reactor as in Example 1, the reaction was carried out on the same scale except that the refrigerant of -15°C was used. The temperature at the top of the reactor was 2.0°C, and the temperature at the bottom of the reactor was 0°C. After the reaction reached a steady state, the reaction solution was analyzed, revealing that conversion of allyl alcohol was 100%, yield of 2,3-DCP was 94.1%, the amount of by-product monochlorohydrin was 3.6%, and the amount of 1,2,3-trichloropropane was 0.5%.

COMPARATIVE EXAMPLE 2

Using the same reactor as in Comparative Example 1, the refrigerant temperature was adjusted and the reaction was performed on the same scale so that the reaction temperature became 2.0°C. After the reaction reached a steady state, the reaction solution was analyzed, revealing that conversion of allyl alcohol was 100%, yield of 2,3DCP was 92.6%, the amount of by-product monochlorohydrin was 3.6%, and the amount of by-product 1,2,3-trichloropropane: 1.0%.

As stated above, the process for producing 2,3DCP in accordance with this invention makes it possible to increase the hydrogen chloride concentration of hydrochloric acid used as a solvent and to reduce the residence time of reaction liquid in the reactor, whereby formation of both by-products due to hypochlorous acid and by-products based on chlorination can be inhibited, and the yield of 2,3DCP can be increased.

**Claims**

1. A process for producing 2,3-dichloro-1-propanol, which comprises reacting allyl alcohol dissolved in hydrochloric acid with chlorine using a falling film type reactor.

2. A chlorination reactor for the production of 2,3-dichloro-1-propanol by reacting allyl alcohol dissolved in hydrochloric acid with chlorine, which comprises:

   (i) a cylindrical shell (2) having

      (a) nozzles (3) and (4) for introducing and discharging a refrigerant provided in lower and upper portions of the shell,
      (b) a horizontal upper tube sheet (5) and a lower tube sheet (6), and
      (c) a plurality of vertical falling film tubes (7) provided between the upper and lower tube sheets (5,6), through which a portion above the upper tube sheet (5) is connected to a portion beneath the lower tube sheet (6);

   (ii) a lower cover (13) beneath the shell (2), having provided therein

      (d) a nozzle (15) for discharging a reaction liquid and a nozzle (16) for introducing a starting material; and

   (iii) an upper cover (25) above the shell (2), having provided therein

      (e) a horizontal restriction plate (21) separating the upper cover (25) into a lower liquid reservoir (22) having a liquid-introducing nozzle (26) and an upper gas reservoir (23) having a gas-introducing nozzle (27); characterized in that the upper surface of the upper tube sheet (5) is provided with
      (f) liquid distribution portions (28) of the falling film tubes (7) pierced through the upper tube sheet (5), the liquid distribution portions having holes (32) in the vicinity of the upper surface of the upper tube sheet (5) to allow a liquid in the liquid reservoir to come into the falling film tubes (7) and having an upper end opening close to the restriction plate (21) which has formed therein an orifice hole (30) facing toward the upper end opening of the falling film tube (7) to allow a gas in the gas reservoir (23) to be uniformly distributed in each of the falling film tubes (7).

**Patentansprüche**

1. Verfahren zur Herstellung von 2,3-Dichlor-1-propanol durch Umsetzen von in Salzsäure gelöstem Allylalkohol mit Chlor unter Verwendung eines Reaktors vom Fallfilmtyp.

2. Chlorierungsreaktor für die Herstellung von 2,3-Dichlor-1-propanol durch Umsetzen von in Salzsäure gelöstem Allylalkohol mit Chlor, umfassend:

   (i) einen zylindrischen Mantel (2) mit

      (a) in unteren und oberen Bereichen des Mantels vorgesehenen Rohrstutzen (3) bzw. (4) zum Einführen und Abführen eines Kältemittels,
      (b) einer waagerechten oberen Rohrplatte (5) und einer (waagerechten) unteren Rohrplatte (6) sowie
      (c) mehreren zwischen den oberen und unteren Rohrplatten (5, 6) angeordneten lotrechten Fallfilmrohren (7), durch welche ein Bereich über der oberen Rohrplatte (5) mit einem Bereich unter der unteren Rohrplatte (6) verbunden ist,

   (ii) eine unterhalb des Mantels (2) angeordnete untere Haube (13), in welcher

(d) ein Rohrstutzen (15) zum Austragen einer Reaktionsflüssigkeit und ein Rohrstutzen (16) zum Einführen eines Ausgangsmaterials vorgesehen sind, sowie

(iii) eine oberhalb des Mantels (2) angeordnete obere Haube (25), in welcher

(e) eine waagerechte Trennplatte (21) angeordnet ist, welche die obere Haube (25) in ein unteres Flüssigkeitsreservoir (22) mit einem Flüssigkeitseinführrohrstutzen (26) und ein oberes Gasreservoir (23) mit einem Gaseinführrohrstutzen (27) unterteilt,
dadurch gekennzeichnet, daß die Oberseite der oberen Rohrwand (5) mit
(f) Flüssigkeitsverteilungsabschnitten (28) der die obere Rohrwand (5) durchsetzenden Fallfilmrohre (7) versehen ist, welche Flüssigkeitsverteilungsabschnitte in der Nähe der Oberseite der oberen Rohrwand (5) Öffnungen (32), um einer im Flüssigkeitsreservoir befindlichen Flüssigkeit einen Eintritt in die Fallfilmrohre (7) zu gestatten, und eine obere Endöffnung dicht an der Trennplatte (21) aufweisen, in welcher Trennplatte eine zur oberen Endöffnung des Fallfilmrohrs (7) weisende Düsenöffnung (30) zur Ermöglichung einer gleichmäßigen Verteilung eines im Gasreservoir (23) befindlichen Gases in die jeweiligen Fallfilmrohre (7) ausgebildet ist.

## Revendications

1. Procédé de production de 2,3-dichloro-1-propanol qui consiste à faire réagir de l'alcool allylique dissous dans de l'acide chlorhydrique avec du chlore en utilisant un réacteur de type à film ruisselant.

2. Réacteur de chloration pour la production de 2,3-dichloro-1-propanol par réaction d'alcool allylique dissous dans de l'acide chlorhydrique avec du chlore qui comprend :

(i) un boîtier cylindrique (2) présentant

(a) des buses (3) et (4) pour introduire et évacuer un réfrigérant fourni dans des parties inférieure et supérieure du boîtier,
(b) une plaque tubulaire supérieure horizontale (5) et une plaque tubulaire inférieure (6), et
(c) de nombreux tubes verticaux à film ruisselant (7) fournis entre les plaques tubulaires supérieure et inférieure (5,6) par l'intermédiaire desquels une partie se trouvant au-dessus de la plaque tubulaire supérieure (5) est raccordée à une partie se trouvant en dessous de la plaque tubulaire inférieure (6) ;

(ii) un couvercle inférieur (13) en dessous du boîtier (2) présentant à l'intérieur de celui-ci

(d) une buse (15) pour évacuer un liquide de réaction et une buse (16) pour introduire un matériau de départ ; et

(iii) un couvercle supérieur (25) au-dessus du boîtier (2) présentant à l'intérieur de celui-ci

(e) une plaque de restriction horizontale (21) séparant le couvercle supérieure (25) en un réservoir inférieur de liquide (22) présentant une buse d'introduction de liquide (26) et un réservoir supérieur de gaz (23) présentant une buse d'introduction de gaz (27) ;caractérisé en ce que la surface supérieure de la plaque tubulaire supérieure (5) est munie
(f) de parties de distribution de liquide (28) des tubes à film ruisselant (7) percées à travers la plaque tubulaire supérieure (5), les parties de distribution de liquide présentant des trous (32) au voisinage de la surface supérieure de la plaque tubulaire supérieure (5) pour permettre à un liquide dans le réservoir de liquide d'arriver dans les tubes à film ruisselant (7) et présentant une ouverture d'extrémité supérieure proche de la plaque de restriction (21) qui a formé dans celle-ci un trou d'embouchure (30) faisant face à l'ouverture d'extrémité supérieure du tube à film ruisselant (7) pour permettre à un gaz dans le réservoir de gaz (23) d'être uniformément distribué dans chacun des tubes à film ruisselant (7).

Fig. 1

Fig. 2

Fig. 3

Fig. 4

Fig. 5

Fig. 7

Fig. 6

Fig. 8